# EUROPÄISCHE PATENTSCHRIFT

(11) **EP 0 731 081 B1**
(45) Veröffentlichungstag und Bekanntmachung des Hinweises auf die Patenterteilung: **15.04.1998**
(21) Anmeldenummer: 96103321.4
(22) Anmeldetag: 04.03.1996
(51) Int. Cl.: C07C 53/21, C07C 51/42, C07C 51/41

(54) **Rückgewinnung hochfluorierter Carbonsäuren aus der Gasphase**
Recovery of highly fluorinated carboxylic acids from gaseous phase
Récupération d'acides carboxyliaues à un haut degré fluorés à partir d'une phase gazeuse

(30) Priorität: 09.03.1995 DE 19508447; 26.07.1995 DE 19527276
(43) Veröffentlichungstag der Anmeldung: 11.09.1996
(73) Patentinhaber: Dyneon GmbH, 84504 Burgkirchen (DE)
(72) Erfinder: Sulzbach, Reinhard A., Dr., D-84489 Burghausen (DE); Kowatsch, Wilhelm, D-84518 Garching/Alz (DE); Steidl, Dieter, D-65719 Hofheim (DE)

(56) Entgegenhaltungen:
- FR-A- 814 839
- GB-A- 1 127 521
- US-A- 2 516 127

## Beschreibung

Bei der Polymerisation fluorierter Monomerer in wäßriger Dispersion werden hochfluorierte Carbonsäuren als Emulgatoren eingesetzt. Hauptsächlich kommen hierfür perfluorierte Alkancarbonsäuren mit 7 bis 10 Kohlenstoffatomen, insbesondere die Perfluoroctansäure, in Form ihrer leichtlöslichen Salze, bevorzugt Ammoniumsalze, in Betracht.

Gewinnt man die Fluorpolymeren aus dem primär entstehenden Fluorpolymerlatex durch Koagulation, so verbleiben die hochfluorierten Carbonsäuren zu einem erheblichen Teil im sich bildenden Fluorpolymerpulver. In Abhängigkeit vom pH-Wert, bei dem der Koagulationsprozeß durchgeführt wird, können die hochfluorierten Carbonsäuren entweder als freie Säuren oder aber in Form ihrer Ammoniumsalze vorliegen. Auch Gemische aus freien Säuren und Ammoniumsalzen sind möglich. Die Ammoniumsalze der hochfluorierten Carbonsäuren besitzen bei den üblichen Trocknungstemperaturen für Fluorpolymerpulver im Bereich zwischen 120 und 300 °C einen erheblichen Dampfdruck, so daß sie zusammen mit dem verdampfenden Wasser bei der Trocknung des Pulvers mit Luft freigesetzt werden und sich dann meistens in sehr niedriger Konzentration in den Abgasen beziehungsweise der Abluft des Trockners befinden. Freie hochfluorierte Carbonsäuren bilden mit Wasser niedrigsiedende Azeotrope, so daß eine weitgehend quantitative Abtrennung aus dem Fluorpolymerpulver in die Gasphase möglich ist.

Zahlreiche Fluorpolymere werden heute großtechnisch nach dem Verfahren der Emulsionspolymerisation hergestellt. Hierzu zählen: Emulsionspolymerisate des Tetrafluorethylens, die mit geringen Mengen an Hexafluorpropylen und/oder Perfluorpropylvinylether modifiziert sein können und die als sogenannte Pastenprodukte nicht aus der Schmelze verarbeitbar sind, Copolymerisate aus Tetrafluorethylen und Hexafluorpropylen beziehungsweise Perfluorpropylvinylether, Copolymerisate aus Tetrafluorethylen, Ethylen und gegebenenfalls weiteren Comonomeren, und Copolymerisaten des Vinylidenfluorids mit weiteren Comonomeren wie Tetrafluorethylen und Hexafluorpropylen, um nur die wesentlichen Beispiele zu nennen.

Bei der Aufarbeitung all dieser Fluorpolymeren kann das erfindungsgemäße Verfahren Anwendung finden. Ganz allgemein gesehen ist das erfindungsgemäße Verfahren immer dann anwendbar, wenn hochfluorierte Alkancarbonsäuren in Abluft- oder Abgasströmen vorhanden sind und aus diesen abgetrennt werden sollen. Es ist auch dann gut anwendbar, wenn die hochfluorierten Carbonsäuren in sehr niedriger Konzentration vorliegen.

Hochfluorierte Carbonsäuren sind einerseits teure Substanzen und andererseits sind sie biologisch nicht abbaubar, so daß sie aus Kostengründen und aus Gründen des Umweltschutzes möglichst quantitativ zurückgewonnen werden müssen.

Die Beseitigung der hochfluorierten Carbonsäuren aus Abgasen beziehungsweise Abluft durch Auswaschen mit Wasser ist unbefriedigend, da im Falle der freien Säuren aufgrund des hohen Dampfdruckes keine hinreichend gute Reinigung erfolgt und so die Effizienz des Waschprozesses unzureichend ist. Im Falle der Ammoniumsalze erhält man bei relativ niedriger Anreicherung in der Waschlösung bereits eine Abscheidung von Ammoniumsalzen, was die Aufkonzentration auf höhere Gehalte erschwert.

Die Beseitigung der hochfluorierten Carbonsäuren aus Abgasen durch Auswaschen mit verdünnten Laugen stößt in der Praxis ebenfalls auf große Schwierigkeiten, da die Säuren und insbesondere ihre Salze als stark oberflächenaktiv wirkende Substanzen hierbei zu einer starken Schaumbildung führen. Die technische Rückgewinnung dieser wertvollen Substanzen ist daher auf diesem Wege ebenfalls sehr erschwert. Der Zusatz üblicher Entschäumer löst diese Schwierigkeiten nicht.

Es wurde nun gefunden, daß hochfluorierte Carbonsäuren beziehungsweise deren Ammoniumsalze aus Abgasströmen dann gut ausgewaschen werden können, wenn man eine alkalische Waschlösung hoher Dichte einsetzt, nämlich entweder konzentrierte Laugen verwendet oder den verdünnteren Laugen Salze, vorzugsweise Alkalisalze, zusetzt. Dadurch wird das Schäumen unterdrückt. Außerdem wird die Löslichkeit der sich bildenden Salze der hochfluorierten Carbonsäuren im Waschmedium stark herabgesetzt, so daß sich die Salze der Fluorcarbonsäuren als zweite Phase aus dem alkalischen Waschmedium in konzentrierter Form abscheiden und so einfach aus dem alkalischen Waschmedium abgetrennt werden können.

Es ist für das erfindungsgemäße Verfahren unerheblich, ob die hochfluorierten Carbonsäuren in freier Form oder in Form ihrer Ammoniumsalze in den zu reinigenden Abgasen vorliegen, da die Ammoniumsalze in der alkalischen Waschlösung ebenfalls in die entsprechenden Alkalisalze umgewandelt werden.

Die Temperatur des Waschprozesses ist nicht kritisch. Sie kann etwa 10 bis 80 °C, vorzugsweise 20 bis 50 °C, betragen. Bei tieferen Temperaturen sind die Löslichkeitsgrenzen von Alkalilauge beziehungsweise Alkalisalz zu beachten. Die Waschlösung als solche soll eine echte Lösung sein. Es darf zu keiner Auskristallisation der Alkalilauge beziehungsweise des Alkalisalzes kommen.

Das erfindungsgemäße Verfahren kann sowohl bei einem Druck unter 1 bar absolut, bei Atmosphärendruck oder mehreren bar Überdruck betrieben werden.

In einer bevorzugten Ausführungsform wird die Dichte der alkalischen Waschlösung auf einen Wert eingestellt, der höher ist als die Dichte der sich abscheidenden Salze der Fluorcarbonsäuren, so daß sich diese in einem Absetzbehälter als obere Phase auf der Waschlösung hoher Dichte abscheiden und ausgeschleust werden. Die Waschlösung wird unten abgezogen und direkt in den Waschprozeß zurückgeführt. Die Dichte der alkalischen Waschlösung soll über 1,15 g/cm³, vorzugsweise über 1,3 g/cm³, bei der entsprechenden Temperatur im Wäscher betragen. Die im Kreis geführte Waschlösung enthält unter 1 %, wobei hier und im folgenden die Prozentangaben sich auf das Gewicht beziehen, im allgemeinen unter 0,1 % an Salzen der hochfluorierten Carbonsäuren.

Ein störendes Schäumen tritt beim Waschprozeß unter diesen Bedingungen nicht ein. Die ausfallenden Salze der hochfluorierten Carbonsäuren stören den Waschprozeß nicht, da sie unmittelbar nach ihrer Entstehung in einem Abscheidebehälter abgeschieden werden.

Zweckmäßig wählt man als alkalische Verbindung ein Alkalihydroxid, vorzugsweise Kalilauge und insbesondere Natronlauge, wobei die Konzentration so bemessen wird, daß die Dichte über 1,15 g/cm³ liegt. Bei Kalilauge ist dies bei einer Konzentration von über 16 % und bei Natronlauge bei einer Konzentration von über 14 % der Fall. Auch Mischungen verschiedener Alkalien sind möglich.

Möchte man mit niedrigeren Konzentrationen an Alkalihydroxid arbeiten, kann die Dichte der Waschlösung von > 1,15 g/cm³ auch durch Zusatz eines Salzes erreicht werden. Als Salze eignen sich ganz allgemein anorganische Verbindungen, die im alkalischen Milieu keine schwerlöslichen Hydroxide bilden. Dies sind insbesondere Alkalisalze wie Natrium- oder Kaliumchlorid, -bromid oder -sulfat. Da Chloridionen jedoch bei Verwendung von Metallen als Werkstoff für die Aufarbeitungsanlagen Korrosion verursachen können, werden andere Salze, wie beispielsweise Sulfate, zur Einstellung der Dichte der Waschlösung bevorzugt. Vorteilhaft wählt man ein Salz mit dem gleichen Kation wie die alkalische Verbindung, beim Einsatz von Natronlauge also vorzugsweise Natriumsulfat. Auch Mischungen von Salzen sind möglich.

Die leichtere, die Salze der hochfluorierten Carbonsäuren enthaltende obere Phase fällt in Form eines Salzbreies an, dem noch alkalisches wäßriges Medium, also noch Alkalihydroxid und gegebenenfalls auch das zugesetzte Salz, anhaftet.

Im Falle der Rückgewinnung von Perfluoroctansäure besteht beim bevorzugten Einsatz von Natronlauge die wertstoffhaltige obere Phase aus:
- etwa 5 bis etwa 20 % Natriumsalz der Perfluoroctansäure,
- unter 10 % Natriumhydroxid und
- Wasser, ergänzt auf 100 %.

Beim Einsatz von Natronlauge und Natriumsulfat besteht die wertstoffhaltige obere Phase aus:
- etwa 30 bis etwa 55 % Natriumsalz der Perfluoroctansäure,
- unter 10 %, vorzugsweise unter 5 %, Natriumhydroxid,
- etwa 20 bis 40 % Natriumsulfat und
- Wasser, ergänzt auf 100 %.

In der oberen wertstoffhaltigen Phase kann auch zusätzlich Natriumcarbonat vorhanden sein, wenn der zu reinigende Abgasstrom Kohlendioxid enthält.

Diese hier angegebenen Zusammensetzungen sind als beispielhaft zu verstehen. Sie werden beeinflußt von der vorgegebenen Konzentration an Alkalihydroxid beziehungsweise von der Konzentration des zugegebenen Salzes in der Waschlösung.

Sammelt man die abgeschiedene Oberphase, zum Beispiel in einem Tank, so stellt man fest, daß nach mehrstündigem Stehen eine weitere Anreicherung der Salze der hochfluorierten Carbonsäuren stattfindet, indem sich erneut wenigstens zwei Phasen ausbilden. Man stellt durch Analyse fest, in welcher Phase die Salze der hochfluorierten Carbonsäuren enthalten sind und führt die anderen Phasen vorzugsweise in den Prozeß zurück.

Unter gewissen Bedingungen wird der angereicherte Salzbrei der hochfluorierten Carbonsäuren fest. Der Fachmann wird dann im Einzelfall abwägen, ob er im Hinblick auf seine apparativen Möglichkeiten bei der Weiterverarbeitung eine höhere oder niedrigere Konzentration an Salzen der hochfluorierten Carbonsäuren wünscht.

Die Weiterverarbeitung der Salze der hochfluorierten Carbonsäuren zu einer in der Polymerisation von Fluormonomeren wiederverwendbaren Salzlösung, vorzugsweise einer Ammoniumsalzlösung, erfordert weitere Reinigungsschritte. Hierbei kann beispielsweise nach dem in der US-A 5 442 097 beschriebenen Verfahren der Veresterung gearbeitet werden.

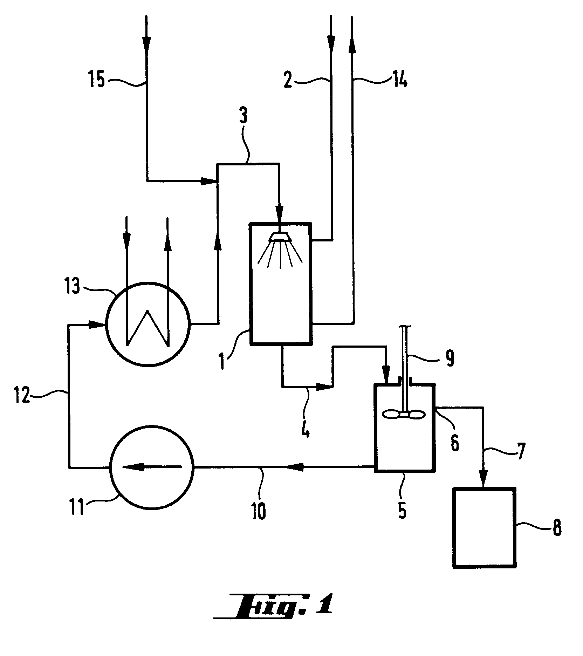

Zur weiteren Erläuterung des erfindungsgemäßen Verfahrens wird eine kontinuierliche Rückgewinnung von Perfluoroctansäure anhand der Figur 1 näher erläutert.

Wesentlicher Bestandteil der Anlage ist der Wäscher 1, in dem die perfluoroctansäurehaltige Abluft über die Leitung 2 mit der alkalischen Waschlösung hoher Dichte, zugeführt durch die Leitung 3, in innigen Kontakt gebracht wird. Hierbei bildet sich das in der alkalischen Waschlösung schwer lösliche Alkalisalz der Perfluoroctansäure und wird als feste Phase ausgeschieden. Das heterogene Gemisch aus alkalischer Waschlösung und ausgeschiedenem Alkalisalz der Perfluoroctansäure strömt über die Leitung 4 in den Abscheidebehälter 5. Hier trennt sich nun in einer beruhigten Zone des Behälters das spezifisch leichtere Salz der Perfluoroctansäure als Oberphase von der schwereren alkalischen Waschlösung in Form eines Salzbreies ab. Der entstehende Salzbrei wird über einen Überlauf 6 am Behälter, zusammen mit der anhaftenden alkalischen Waschlösung, über die Leitung 7 ausgetragen und läuft kontinuierlich in einen Sammelbehälter 8 ab. Der Austrag des Salzbreis kann durch ein Förderorgan 9, beispielsweise einem sehr langsam laufenden Rührer, begünstigt werden. Die Höhe des Rührers 9 wird dabei so eingestellt, daß er gerade in den Behälterinhalt eintaucht und dadurch den Abscheideprozeß im Behälter nicht stört.

In einer bevorzugten Ausführungsform wird das als obere Phase im Sammelbehälter 8 abgeschiedene rohe Salz der Perfluoroctansäure mit 1 Gew.-Teil Wasser pro 2 bis 5 Gew.-Teile Salz versetzt, innig durchmischt und die sich bildenden Phasen neuerlich getrennt.

Die alkalische Waschlösung hoher Dichte wird aus dem unteren Teil des Abscheidebehälters 5 über die Leitung 10 mit der Pumpe 11 über die Leitung 12 durch einen Wärmetauscher 13 und die Leitung 3 der Waschkolonne 1 wieder zugeführt. Die gereinigte Abluft wird über die Leitung 14 abgeführt. Die im Kreis geführte Waschlösung enthält aufgrund der geringen Löslichkeit keine nenneswerten Mengen an Alkalisalz der Perfluoroctansäure mehr. Die alkalische Waschlösung wird über die Leitung 15 in dem Maße mit Lauge und gegebenenfalls mit Salz ergänzt, wie es dem Austrag an diesen Komponenten am Überlauf des Behälters 5 entspricht. Dadurch wird die erforderliche hohe Dichte der Waschlösung aufrechterhalten. Die Ergänzung der Komponenten der Waschlösung kann kontinuierlich oder portionsweise erfolgen.

Außerdem ist die Bilanz an Wasser zu berücksichtigen: Wasserdampf wird meist mit den perfluoroctansäurehaltigen Abgasen, die aus Trocknungsprozessen stammen, in den Wäscher 1 eingetragen. Hierbei kondensieren die Dämpfe im Waschmedium hoher Dichte und setzen die Konzentration herab. Andererseits ist die gereinigte Abluft, die den Wäscher 1 verläßt, mit Wasser gesättigt, und etwas Wasser wird über den Behälter 5 ausgetragen. Um die Konzentration des Waschmediums aufrechtzuerhalten, empfiehlt es sich, die Dichte kontinuierlich zu messen und danach Alkalilauge, gegebenenfalls Salz und Wasser in den erforderlichen Mengen nachzuspeisen.

Die aus dem Wäscher 1 entweichende, gereinigte Abluft enthält nur noch sehr geringe Mengen an Perfluoroctansäure beziehungsweise an Ammoniumsalz der Perfluoroctansäure. Es werden Werte erreicht, die unter 5 mg/Nm³, vorzugsweise unter 1 mg/Nm³, Abgas liegen.

Die Erfindung wird nun anhand der folgenden Beispiele noch näher erläutert. Auch hier beziehen sich die Prozentangaben auf das Gewicht.

### Beispiel 1

In eine handelsübliche Waschkolonne mit einer Länge von 2000 mm und einem Innendurchmesser von 250 mm werden 400 Nm³/h Abluft mit einer Temperatur von 171 °C aus einem Trocknungsprozeß für Fluorpolymerpulver eingetragen. Die Abluft enthält 750 mg/Nm³, entsprechend 300 g/h, Perfluoroctansäure. Mit der Abluft werden zusätzlich circa 20 kg/h Wasser aus dem Trockner in den Waschprozeß transportiert. Der Druck in der Waschkolonne beträgt circa 1 bar absolut.

Dem Wäscher werden über eine Düse 10 m³/h einer alkalischen Waschflüssigkeit, im wesentlichen bestehend aus wäßriger Natronlauge mit einer Dichte von 1,34 g/cm³ und einer Temperatur von 45 °C, aufgegeben. Am unteren Teil der Waschkolonne entweicht der gereinigte Abgasstrom von 400 Nm³/h, der nur noch 0,8 mg/Nm³, entsprechend 0,32 g/h Perfluoroctansäure enthält. Zusätzlich ist Wasserdampf, entsprechend dem Wasserpartialdruck, bei der im Wäscher herrschenden Temperatur von circa 45 °C vorhanden.

Der Wäscher wird im Gleichstrom betrieben. Das alkalische Waschmedium und das entstandene Natriumsalz der Perfluoroctansäure strömen aus der Kolonne direkt in einen Abscheidebehälter mit einem Volumen von 0,4 m³. Dort schwimmt das in der alkalischen Waschlösung nicht lösliche Natriumsalz der Perfluoroctansäure als breiige Schicht auf. Die alkalische Waschlösung, die praktisch kein Natriumsalz der Perfluoroctansäure mehr enthält, wird unten aus dem Abscheidebehälter abgezogen und mit einer Pumpe über einen Wärmetauscher der Waschkolonne erneut zugeführt. Die Konzentration an gelöstem Natriumsalz der Perfluoroctansäure im Waschmedium beträgt etwa 20 mg/l. Die Dichte der Waschflüssigkeit wird durch Zugabe von Natronlauge auf dem gewünschten Wert von etwa 1,34 g/cm³ aufrechterhalten. Das als breiige Schicht abgeschiedene Natriumsalz der Perfluoroctansäure läuft an einem Überlauf des Abscheidebehälters zusammen mit etwas Waschmedium in einen Tank ab. Der Austragsvorgang wird durch einen sehr langsam laufenden Rührer unterstützt, der gerade in die obere Schicht eintaucht.

In dem Tank scheiden sich nach mehrstündigem Stehen nochmals zwei Phasen ab, eine Unterphase, die im wesentlichen aus überschüssiger Waschflüssigkeit besteht, und eine breiige Oberphase. Die Unterphase wird abgetrennt und in den Waschprozeß zurückgeführt.

Die breiige, das Natriumsalz der Perfluoroctansäure enthaltende Oberphase wird zur Herabsetzung des Natronlaugengehaltes und zur besseren Handhabung folgender Prozedur unterzogen:
- Zugabe von 25 kg Wasser pro 100 kg Oberphase unter Rühren,
- Phasentrennung nach mehrstündigem Stehen,
- Abtrennung der Unterphase und Rückführung in den Waschprozeß nach Aufkonzentration mit Natronlauge,
- neuerliche Zugabe von 50 kg Wasser pro 100 kg Oberphase unter Rühren und
- Probenahme unter Rühren.

Die auf diese Art gewonnene Probe besitzt folgende Zusammensetzung:
- 21,9 %: Natriumperfluoroctanoat
- 8,9 %: Natriumhydroxid
- 3,6 %: Natriumcarbonat
Rest Wasser

Das so gewonnene Konzentrat wird der weiteren Aufarbeitung zur Gewinnung reiner 100%iger Perfluoroctansäure zugeführt.

Es sei erwähnt, daß das so gewonnene Konzentrat zwar eine rühr- und pumpfähige Mischung, aber keine stabile Lösung des Natriumsalzes der Perfluoroctansäure darstellt. Um eine stabile Lösung zu erhalten, müßte noch mehr Wasser zugesetzt werden. Dies ist für die weitere Aufarbeitung jedoch nicht unbedingt erforderlich.

Durch die beschriebene Rückführung der abgetrennten Unterphase in den Waschprozeß geht keine Perfluoroctansäure verloren.

### Beispiel 2

Es wird analog Beispiel 1 gearbeitet. Zum Unterschied wird jedoch als Waschlösung ein Gemisch aus 5 % Natriumhydroxid, 19 % Natriumsulfat und Rest im wesentlichen Wasser eingesetzt. Die umgepumpte Menge beträgt 6 m³/h bei 26 °C. Die Dichte des Waschmediums beträgt 1,24 g/cm³. Der eingetragene Abgasstrom beträgt 400 Nm³/h. Er enthält 770 mg/Nm³, entsprechend 308 g/h Perfluoroctansäure. Am unteren Teil der Kolonne entweicht der gereinigte Abgasstrom, der noch 3 mg/Nm³, entsprechend 1,2 g/h Perfluoroctansäure enthält. Die im Abscheidebehälter aufschwimmende, das Natriumsalz der Perfluoroctansäure enthaltende Phase zeigt folgende Zusammensetzung:
- 34,0 %: Natriumperfluoroctanoat
- 3,5 %: Natriumhydroxid
- 20,0 %: Natriumsulfat
- 2,1 %: Natriumcarbonat
Rest Wasser

## Patentansprüche

1. Verfahren zur Rückgewinnung hochfluorierter Carbonsäuren beziehungsweise deren Ammoniumsalze aus Abgasströmen, dadurch gekennzeichnet, daß man das Abgas mit einer so konzentrierten alkalischen Waschlösung in Kontakt bringt, daß sich das Salz der hochfluorierten Carbonsäure als separate Phase abscheidet.

2. Verfahren nach Anspruch 1, dadurch gekennzeichnet, daß die alkalische Waschlösung eine Dichte > 1,15 g/cm³ hat.

3. Verfahren nach Anspruch 1, dadurch gekennzeichnet, daß die alkalische Waschlösung eine Dichte > 1,3 g/cm³ hat.

4. Verfahren nach einem oder mehreren der vorhergehenden Ansprüche, dadurch gekennzeichnet, daß die alkalische Waschlösung eine Alkalilauge ist, die ein Salz enthalten kann.

5. Verfahren nach Anspruch 4, dadurch gekennzeichnet, daß das Salz ein Alkalisalz ist, das das gleiche Kation wie die Alkalilauge enthält.

6. Verfahren nach Anspruch 4, dadurch gekennzeichnet, daß die Alkalilauge Natron- oder Kalilauge ist.

7. Verfahren nach einem oder mehreren der vorhergehenden Ansprüche, dadurch gekennzeichnet, daß sich das Salz der hochfluorierten Carbonsäure auf der Waschlösung als obere Phase abscheidet.

8. Vorrichtung zur Durchführung des Verfahrens nach einem oder mehreren der vorhergehenden Ansprüche, gekennzeichnet durch einen Wäscher (1), eine Zuführung (2) für das zu reinigende Abgas, eine Zuführung (3) für die Waschlauge, einen über eine Leitung (4) verbundenen Abscheidebehälter (5), der einen Überlauf (6) enthält, von dem eine Leitung (7) in einen Sammelbehälter (8) führt, sowie eine Abgasleitung (14).

9. Vorrichtung nach Anspruch 8, gekennzeichnet durch eine Kreislaufleitung (10, 12, 3), die über eine Pumpe (11) und einen Wärmetauscher (13) den Abscheidebehälter (5) mit dem Wäscher (1) verbindet.

10. Vorrichtung nach Anspruch 8 oder 9, dadurch gekennzeichnet, daß der Abscheidebehälter (5) ein Förderorgan (9) enthält.

## Claims

1. A process for the recovery of highly fluorinated carboxylic acids or their ammonium salts from exhaust gas streams, which comprises bringing the exhaust gas into contact with an alkaline scrubbing solution of a concentration such that the salt of the highly fluorinated carboxylic acid separates out as a separate phase.

2. The process as claimed in claim 1, wherein the alkaline scrubbing solution has a density > 1.15 g/cm³.

3. The process as claimed in claim 1, wherein the alkaline scrubbing solution has a density > 1.3 g/cm³.

4. The process as claimed in one or more of the preceding claims, wherein the alkaline scrubbing solution is an alkali metal hydroxide solution which can contain a salt.

5. The process as claimed in claim 4, wherein the salt is an alkali metal salt which contains the same cation as the alkali metal hydroxide.

6. The process as claimed in claim 4, wherein the alkali metal hydroxide solution is sodium hydroxide solution or potassium hydroxide solution.

7. The process as claimed in one or more of the preceding claims, wherein the salt of the highly fluorinated carboxylic acid separates out as an upper phase on the scrubbing solution.

8. An apparatus for carrying out the process as claimed in one or more of the preceding claims, which comprises a scrubber (1), a feed (2) for the exhaust gas to be cleaned, a feed (3) for the scrubbing liquor, a separation vessel (5) connected via a line (4), which separation vessel contains an overflow (6) from which a line (7) runs into a collection vessel (8), and an exhaust gas line (14).

9. The apparatus as claimed in claim 8, wherein a circulation line (10, 12, 3) connects the separation vessel (5) to the scrubber (1) via a pump (11) and a heat exchanger (13).

10. The apparatus as claimed in claim 8 or 9, wherein the separation vessel (5) contains a conveying element (9).

## Revendications

1. Procédé de récupération d'acides carboxyliques hautement fluorés, respectivement de leurs sels d'ammonium à partir d'effluents gazeux, caractérisé en ce que l'on met en contact l'effluent gazeux avec une solution de lavage alcaline tellement concentrée que le sel de l'acide carboxylique hautement fluoré se sépare comme phase séparée.

2. Procédé selon la revendication 1, caractérisé en ce que la solution de lavage alcaline présente une densité >1,15 g/cm³.

3. Procédé selon la revendication 1, caractérisé en ce que la solution de lavage présente une densité >1,3 g/cm³.

4. Procédé selon une ou plusieurs des revendications précédentes, caractérisé en ce que la solution de lavage alcaline est une lessive alcaline pouvant contenir un sel.

5. Procédé selon la revendication 4, caractérisé en ce que le sel est un sel alcalin contenant le même cation que la lessive alcaline.

6. Procédé selon la revendication 4, caractérisé en ce que la lessive alcaline est la lessive de soude ou la lessive de potasse.

7. Procédé selon une ou plusieurs des revendications précédentes, caractérisé en ce que le sel d'acide carboxylique hautement fluoré se sépare de la solution de lavage comme phase supérieure.

8. Dispositif pour la mise en oeuvre du procédé conforme à l'invention selon une ou plusieurs des revendications précédentes, caractérisé en ce qu'il présente un laveur (1), une conduite d'alimentation (2) en effluent gazeux à purifier, une conduite d'alimentation (3) en lessive de lavage, un séparateur (5) relié par une conduite (4), lequel séparateur comporte un trop-plein, depuis lequel part une conduite (7) dans un bac de collecte (8), ainsi qu'une conduite d'effluents gazeux (14).

9. Dispositif selon la revendication 8, caractérisé par une conduite en boucle (10, 12, 3) reliant par l'intermédiaire d'une pompe (11) et d'un échangeur de chaleur (13) le séparateur (5) au laveur (1).

10. Dispositif selon la revendication 8 ou 9, caractérisé en ce que le bac séparateur (5) comporte un organe de transport (9).
